# FASCICULE DE BREVET EUROPEEN

(11) **EP 0 566 442 B1**
(45) Date de publication et mention de la délivrance du brevet: **03.01.1996**
(21) Numéro de dépôt: 93400830.1
(22) Date de dépôt: 31.03.1993
(51) Int. Cl.: A61K 7/027

(54) **Rouge à lèvres mat**
Matter Lippenstift
Matt lipstick

(30) Priorité: 31.03.1992 US 861107
(43) Date de publication de la demande: 20.10.1993
(73) Titulaire: L'OREAL, F-75008 Paris (FR)
(72) Inventeur: Rica, Linda, Kenilworth, New Jersey 07033 (US); Finkenaur, Geoffrey, Clintondale, New York 12515 (US); Miguel, Dolorès, F-92390 Bourg la Reine (FR)
(74) Mandataire: Stalla-Bourdillon, Bernard

(56) Documents cités:
- EP-A- 0 056 219
- EP-A- 0 112 807
- EP-A- 0 195 575
- GB-A- 2 008 943
- US-A- 5 093 111
- PATENT ABSTRACTS OF JAPAN vol. 11, no. 115 (C-415)(2562) 10 Avril 1987

## Description

La présente invention a pour objet une composition de rouge à lèvres et plus particulièrement une composition de rouge à lèvres donnant un aspect mat et ayant une texture homogène et légère ainsi qu'un procédé pour la préparation d'une telle composition de rouge à lèvres.

La fabrication d'un rouge à lèvres sous forme de bâton, résulte d'une pratique relativement nouvelle. Les premiers rouges à lèvres ont été introduits en France et comprenaient un onguent de spermaceti. A l'origine, les rouges à lèvres ne conféraient que très peu de couleur aux lèvres, leur fonction principale étant d'éviter ou d'apaiser les lèvres gercées.

Plus récemment, l'utilisation des bâtons à lèvres comme moyen de coloration des lèvres a subi une croissance rapide, de sorte que les rouges à lèvres sont aujourd'hui des produits de beauté largement répandus. Un rouge à lèvres cosmétiquement accceptable doit s'étaler facilement, être de couleur homogène et présenter un point de fusion supérieur à la température du corps. Par ailleurs, il doit donner aux lèvres un aspect lisse mais non gras et doit garder sa consistance sans que des phénomènes d'exsudation, de suintement, de rupture ou de désagrégation ne se produisent (voir par exemple le document EP-A-0 195 575).

Un rouge à lèvres doit non seulement présenter les qualités mentionnées ci-dessus, mais surtout impartir aux lèvres une sensation lisse et crémeuse et les protéger d'un déssèchement ou de gerçures. Il est notamment difficile d'obtenir une telle sensation lisse et crémeuse à l'aide des rouges à lèvres ayant un lustre ou brillant réduit, à savoir un aspect mat.

Le document GB-A-2 008 943 décrit de microsphères pour la peau d'aspect mat.

De manière courante, les rouges à lèvres mats contiennent diverses argiles et silices en vue d'obtenir un aspect mat. L'emploi d'argiles et de silices conduit à un bâton dur qui devient toujours plus sec et dur lorsque la teneur en substances solides augmente. Par ailleurs, lors de l'application sur les lèvres d'un tel rouge à lèvres mat on obtient une sensation de sécheresse. Il en résulte donc un certain besoin de réaliser un rouge à lèvres qui assure une application crémeuse tout en permettant un aspect mat, ce qui jusqu'à présent était indissociable d'une texture sèche.

La présente invention a pour premier objet de fournir une composition de rouge à lèvres ayant un aspect mat et une texture homogène et légère. La présente invention est caractérisée par le fait que la composition de rouge à lèvres mate comprend une base de rouge à lèvres, ainsi que des sphères d'un copolymère de polyvinylidène en tant qu'agent matifiant efficace. La composition de rouge à lèvres présente de manière avantageuse un aspect mat et une texture homogène et crémeuse.

La présente invention a également pour objet de fournir un procédé pour la préparation d'une composition de rouge à lèvres mate. Cet autre aspect de l'invention est caractérisé par le fait que l'on prépare une composition de rouge à lèvres mate par mélange de sphères d'un copolymère de polyvinylidène à une base de rouge à lèvres. Une composition de rouge à lèvres obtenue selon le procédé de l'invention présente de manière avantageuse un aspect mat et une texture homogène et crémeuse.

La présente invention a donc pour objet une composition de rouge à lèvres mate ayant une texture homogène et légère caractérisée par le fait qu'elle comprend une base de rouge à lèvres et des sphères d'un copolymère de polyvinylidène présentes en faible teneur, en tant qu'agent matifiant efficace. L'emploi de sphères d'un copolymère de polyvinylidène en tant qu'agent matifiant permet l'incorporation dans la composition de rouge à lèvres de quantités plus importantes de substances de nature émolliente sans pour autant aboutir à un effet brillant indésirable.

L'invention a également pour objet un procédé pour la préparation d'une composition de rouge à lèvres mate qui comprend l'addition à une base de rouge à lèvres, d'un agent matifiant à savoir des sphères d'un copolymère de polyvinylidène de manière à obtenir un aspect mat.

La composition de rouge à lèvres selon la présente invention comprend une base de rouge à lèvres et un agent matifiant constitué de sphères d'un copolymère de polyvinylidène. Les sphères du copolymère de polyvinylidène confèrent à la composition de rouge à lèvres non seulement un aspect mat mais également une texture homogène et crémeuse.

Les sphères du copolymère de polyvinylidène sont de préférence sous forme de microsphères creuses ayant une taille de particules comprise entre 5 et 80 µm et une densité spécifique inférieure à 0,2 g/cm³ et de préférence comprise entre 0,01 et 0,065 g/cm³. La partie creuse des microsphères est remplie d'un gaz notamment d'un hydrocarbure tel que l'isobutane ou l'isopentane ou remplie d'air. Les microsphères creuses peuvent être obtenues selon des procédés connus, tels que ceux décrits dans le brevet US N° 3.615.972 et les demandes de brevets européens EP 056.219, EP 112.807, EP 320.472 et EP 486.080. Le copolymère de polyvinylidène utilisé pour les microsphères est une substance thermoplastique non toxique et non irritante telle que les copolymères de chlorure de vinylidène/acrylonitrile et similaires.

Les microsphères creuses du copolymère de polyvinylidène particulièrement préférées sont celles présentant une taille de particules entre 12 et 60 µm et ayant une densité inférieure à 0,1 g/cm³, de préférence comprise entre 0,01 et 0,065 g/cm³ qui sont commercialisées par la Société KEMANORD PLAST sous la marque EXPANCEL et en particulier sous les dénominations de EXPANCEL 551 DE 20 ou EXPANCEL EL 23 (taille de particules d'environ 20 µm) et de EXPANCEL 551 DE (taille de particules d'environ 40 µm). Celles-ci sont des microsphères expansées de copolymère de chlorure de vinylidène/acrylonitrile qui peuvent se présenter à l'état sec ou hydraté (qualité DE ou WE).

Selon l'invention, les microsphères du copolymère de polyvinylidène sont présentes en une proportion comprise entre 0,1 à 1,5% en poids, de préférence entre 0,25 et 0,45% en poids par rapport au poids de la composition de rouge à lèvres.

La composition de rouge à lèvres peut également renfermer en tant qu'agent matifiant complémentaire, un mica enrobé d'une quantité inférieure à 2% d'une silice sphérique. Le mica peut être d'origine naturelle, telle que par exemple la muscovite, la margarite, la roscoélite, la lipidolithe ou d'origine synthétique. Le mica a de préférence une taille de particules inférieure à 15 µm et la silice sphérique a une taille de particules inférieure à 1 µm, de préférence une taille de particules moyenne de 250 nm. Un mica enrobé d'une quantité inférieure à 2%, de préférence entre 1 et 2% d'une silice sphérique est commercialisé sous la dénomination MICRONASPHERE M par la Division Pigments de la Société RONA EM qui est une Société du groupe EM INDUSTRIES. Le mica enrobé d'une quantité inférieure à 2 % de silice est présent dans la composition de rouge à lèvres en une quantité comprise entre 2,0 à 4,0% en poids, de préférence entre 2,5 et 4,0% en poids.

La composition de rouge à lèvres peut également contenir en tant que renforçateur de l'effet matifiant un modificateur de rhéologie tel qu'un argile en particulier les bentonites (quaternium 18 hectorite) en une proportion inférieure à 4% et de préférence inférieure à 2 %.

La base de rouge à lèvres comprend de préférence des ingrédients classiques pour des rouges à lèvres tels que des cires, de préférence les cires de candellila, de paraffine, d'abeilles, d'ozokérite, ou de carnauba, afin de conférer de la dureté et de la rigidité ainsi que d'augmenter le point de fusion, des solvants, des agents liants tels que l'huile de ricin, les esters d'alcools gras ayant de 8 à 10 atomes de carbone, les alcools gras ayant de 10 à 28 atomes de carbone, l'octyldodécanol, l'alcool oléique, l'alcool de lanoline et bien entendu des agents colorants tels que des pigments.

La base de rouge à lèvres peut également comprendre de 0,25 à 1,50% d'une silice fumée d'hydrophobicité accrue telle que le produit commercialisé par la Société DEGUSSA INC. sous la dénomination de AEROSIL R972. L'emploi de cette silice hydrophobe en association avec un modificateur de basse viscosité tel qu'un ester d'acide isononanoïque à raison de 10 à 50%, de préférence l'isononanoate d'isononyle, facilite l'incorporation à un taux très élevé de substances solides, à savoir de 8,0 à 22% en poids dans la composition de rouge à lèvres et améliore la texture et l'obtention d'un effet mat.

La base de rouge à lèvres peut également comprendre de 7 à 10% en poids, de préférence de 8,5 à 9,5% en poids d'amidon octylsuccinate d'aluminium afin d'améliorer la texture et l'obtention d'un effet mat.

En outre, l'emploi dans la base de rouge à lèvres de l'hyaluronate de sodium dans des sphères de silice ayant une taille de particules de 1 à 16 µm et une taille de particules moyenne de 10 µm permet de conférer une forte action humectante. La silice contenant l'hyaluronate de sodium est commercialisée par la Société US COSMETICS CORPORATION Dayville, Connecticut, sous la dénomination commerciale de SB700/HA.

Afin de préparer le rouge à lèvres mat selon l'invention, les pigments sont broyés dans les ingrédients huileux. Ensuite, on procède à la fusion des ingrédients cireux séparément et on les ajoute au mélange précédent sous agitation continue. Après l'addition des autres substances solides, on ajoute sous vide les microsphères creuses du copolymère de polyvinylidène. Enfin, on peut ajouter des parfums appropriés.

Les exemples suivants sont donnés afin d'illustrer l'invention. Ces exemples n'étant présentés qu'à titre d'illustration, l'invention ne peut en aucun cas être limitée à leur objet.

### Exemple 1 :

| | |
|---|---|
| ** Esters d'alcools gras C₈-C₁₀ | 21,0 - 30,0% |
| Alcools gras C₁₀ | 5,0 - 7,0% |
| Amidon octylsuccinate d'aluminium | 7,0 - 10,0% |
| Ozokérite | 7,5 - 10,0% |
| Cire de carnauba | 3,0 - 4,0% |
| Cire d'abeilles | 1,0 - 3,0% |
| Silice colloïdale | 0,2 - 0,7% |
| Sphères de silice contenant de l'hyaluronate de sodium | 0,3 - 1,0% |
| * Copolymère de polyvinylidène | 0,1 - 0,4% |
| Pigment | 6,0 - 9,0% |
| Parfum | 0,1 - 0,5% |
| Huile de ricin | qsp 100 % |

| | |
|---|---|
| * Copolymère de polyvinylidène = EXPANCEL 551 DE 20 | |
| ** Esters d'alcools gras C₈-C₁₀ ayant une viscosité inférieure à 10 cP (10 mPa.s) | |

Le pigment est broyé dans un mélange d'esters d'alcools gras C₈-C₁₀, d'alcools gras C₁₀ et d'huile de ricin. Lors du mélange à l'aide d'un Lightnin Mixer on ajoute la silice colloïdale ainsi que les sphères de silice contenant l'hyaluronate de sodium. L'ozokérite, la cire de carnauba et la cire d'abeilles sont fondues dans un récipient à part et sont introduites dans le mélange sous agitation continue. Ensuite, on introduit sous vide et sous agitation l'amidon octylsuccinate d'aluminium et ensuite le copolymère de polyvinylidène. Enfin, on introduit dans le mélange des parfums.

### Exemple 2

| | |
|---|---|
| Esters d'alcools gras C₈-C₁₀ | 19,0 - 26,0% |
| Alcools gras C₁₀ | 5,0 - 7,0% |
| Amidon octylsuccinate d'aluminium | 7,0 - 10,0% |
| Ozokérite | 7,5 - 10,0% |
| Cire de carnauba | 3,0 - 4,0% |
| * Mica lié avec < 2,0% d'un carbonate de silice sous forme nano-sphérique | 2,0 - 4,0% |
| Cire d'abeilles | 1,0 - 3,0% |
| Silice colloïdale | 0,2 - 0,7% |
| Silice sphérique contenant de l'hyaluronate de sodium | 0,3 - 1,0% |
| Copolymère de polyvinylidène | 0,1 - 0,4% |
| Pigment | 6,0 - 9,0% |
| Parfum | 0,1 - 0,5% |
| Huile de ricin | qsp 100% |

| | |
|---|---|
| * MICRONASPHERE M (<15µm) | |

La composition de l'exemple 2 a été préparée selon la méthode de l'exemple 1 à l'exception près que le mica lié à la silice nano-sphérique a été introduit sous vide après l'introduction de l'amidon octylsuccinate d'aluminium et avant l'introduction du copolymère de polyvinylidène.

### Exemple 3 :

| | |
|---|---|
| ** Esters d'alcools gras C₈-C₁₀ | 25,0% |
| Alcools gras C₁₀ | 6,0 % |
| Amidon octylsuccinate d'aluminium | 7,0 % |
| Ozokérite | 7,5 % |
| Cire de carnauba | 3,0 % |
| Cire d'abeilles | 3,0 % |
| Silice colloïdale | 0,7 % |
| Sphères de silice contenant de l'hyaluronate de sodium | 0,7 % |
| * Copolymère de polyvinylidène | 0,2 % |
| Pigment | 8,0 % |
| Parfum | 0,1 % |
| Bentone (quaternium 18 Hectorite) | 3,0 % |
| Huile de ricin | qsp 100% |

| | |
|---|---|
| * Copolymère de polyvinylidène = EXPANCEL 551 DE 20 | |
| ** Esters d'alcools gras C₈-C₁₀ ayant une viscosité inférieure à 10 cP (10 mPa.s) | |

Ce rouge à lèvres est obtenu selon le même mode opératoire que celui décrit à l'Exemple 1. Il présente une texture homogène et légère et confère aux lèvres un aspect mat.

### Exemple 4

| | |
|---|---|
| Esters d'alcools gras C₈ | 8,0 - 9,0% |
| Isononanoate d'isononyle | 14,0% - 16,0% |
| Stéarate d'octyldodécyle | 6,0% - 7,0% |
| Alcools gras C₁₀ | 5,0 - 7,0% |
| Amidon octylsuccinate d'aluminium | 8,5 - 9,5% |
| Ozokérite | 10,0 - 11,0% |
| Cire de Carnauba | 3,3 - 3,8% |
| * Mica lié avec < 2,0% d'un carbonate de silice | |
| nano-sphérique | 2,5 - 3,5% |
| Cire d'abeilles | 1,5 - 2,5% |
| Silice colloïdale | 0,4 - 0,6% |
| Silice sphérique contenant de l'hyaluronate | |
| de sodium | 0,3 - 0,65% |
| Copolymère de polyvinylidène | 0,1 - 0,4% |
| Pigment | 7,0 - 9,0% |
| Parfum | 0,1 - 0,5% |
| Huile de ricin | qsp 100 % |

La composition de l'exemple 4 a été préparée selon la méthode de l'exemple 2 et a conduit à un rouge à lèvres mat de qualité supérieure. Un rouge à lèvres mat obtenu selon l'exemple 4 a été testé comme suit :

On a donné à 20 personnes un rouge à lèvres obtenu selon l'exemple 4 et on leur a demandé d'utiliser le rouge à lèvres pendant deux jours. Ultérieurement, on a donné aux mêmes personnes un rouge à lèvres mat disponible dans le commerce et on leur a demandé de l'utiliser pendant deux jours. Les nuances testées étaient toutes dans le groupe des rouges et étaient à peu de chose près similaires. A la fin de la période des 4 jours, on a demandé aux personnes de classer les deux rouges à lèvres selon l'échelle numérique donnée ci-dessous :

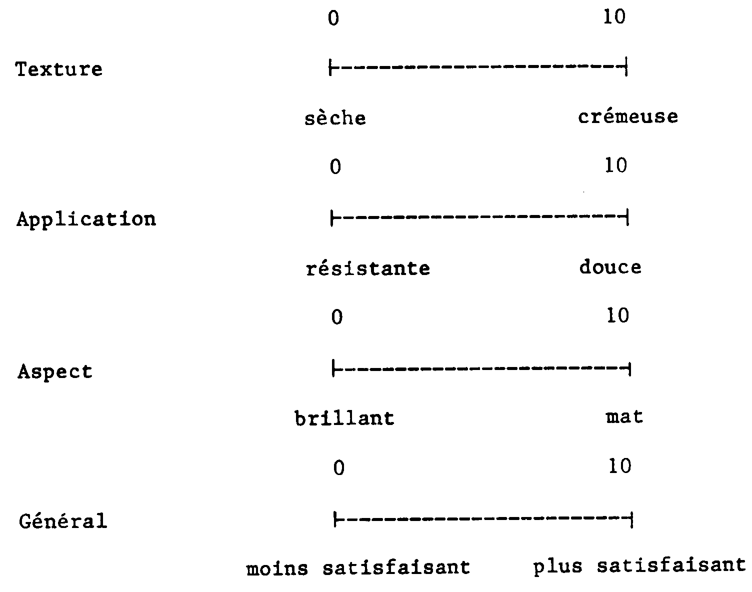

Les résultats de ce test sont présentés ci-dessous au Tableau 1.

**TABLEAU 1**

| Rouge à lèvres | Texture | Application | Aspect | Général | Total |
|---|---|---|---|---|---|
| Exemple 4 | 9 | 8,5 | 9 | 8,5 | 8,75 |
| VELVET TOUCH | 7,5 | 7 | 7,5 | 7,5 | 7,37 |
| MATTE LIPCOLOR | 7 | 6,5 | 6 | 6,5 | 6,50 |
| MATTE | 5 | 4 | 9 | 5 | 5,75 |
| MATTE LE STYLO | 8 | 7 | 8 | 7,5 | 7,62 |

Comme cela ressort du Tableau 1, le rouge à lèvres mat obtenu selon la présente invention présente, par rapport aux produits disponibles dans le commerce, une texture améliorée, une meilleure facilité d'application et un aspect mat supérieur et est donc de manière générale celui qui a été préféré par rapport à tous les produits commerciaux testés.

VELVET TOUCH est un produit commercial de la Société REVLON, MATTE est un produit commercial de la Société SHISEIDO, MATTE LIPCOLOR est un produit commercial de la Société ULTIMA II et MATTE LE STYLO est un produit commercial de la Société LANCOME.

## Revendications

1. Composition de rouge à lèvres mat caractérisée par le fait qu'elle comprend une base de rouge à lèvres et des sphères d'un copolymère de polyvinylidène en une quantité efficace donnant un aspect mat.

2. Composition de rouge à lèvres mat selon la revendication 1, caractérisée par le fait que lesdites sphères du copolymère de polyvinylidène sont présentes en une proportion comprise entre 0,1 et 1,5% en poids par rapport à la composition de rouge à lèvres.

3. Composition de rouge à lèvres mat selon la revendication 1 ou 2, caractérisée par le fait que les sphères du copolymère de polyvinylidène ont une taille de particules de 5 à 80 µm et une densité de 0,01 à 0,065 g/cm³.

4. Composition de rouge à lèvres mat selon l'une des revendications 1 à 3, caractérisée par le fait qu'elle comprend en outre, en tant qu'agent matifiant complémentaire, de 2,0 à 4,0% en poids d'un mica enrobé d'une quantité inférieure à 2% de sphères de silice.

5. Composition de rouge à lèvres mat selon l'une des revendications 1 à 4, caractérisée par le fait qu'elle comprend en outre de 10 à 50% en poids d'un ester d'acide isononanoïque et de 0,25 à 1,50% en poids d'une silice fumée.

6. Composition de rouge à lèvres mat selon l'une des revendications 1 à 5, caractérisé par le fait qu'elle comprend une base de rouge à lèvres, contenant de 10 à 50% en poids d'un ester d'acide isononanoïque et de 0,25 à 1,50% en poids de silice fumée, ainsi que de 2,0 à 4,0% en poids d'un mica enrobé d'une quantité inférieure à 2% de sphères de silice et de 0,1 à 1,5% en poids de sphères d'un copolymère de polyvinylidène, les dites sphères du copolymère de polyvinylidène et le mica enrobé de sphères de silice étant présents en une quantité propre à donner un aspect mat.

7. Composition de rouge à lèvres mat selon la revendication 6, caractérisée par le fait qu'elle comprend de 2,5 à 4,0% en poids d'un mica enrobé d'une quantité inférieure à 2 % de sphères de silice et de 0,25 à 0,45 % en poids d'un copolymère de polyvinylidène.

8. Procédé pour la préparation d'un rouge à lèvres caractérisé par le fait que l'on ajoute à une base de rouge à lèvres un agent matifiant, de façon à donner un aspect mat, ledit agent matifiant comprenant de sphères d'un copolymère de polyvinylidène.

9. Procédé selon la revendication 8, caractérisé par le fait que lesdites sphères du copolymère de polyvinylidène sont présentes en une quantité comprise entre 0,1 à 1,5% en poids, par rapport à la composition de rouge à lèvres.

10. Procédé selon la revendication 8 ou 9, caractérisé par le fait que lesdites sphères du copolymère de polyvinylidène ont une taille de particules de 5 à 80 µm et une densité de 0,01 à 0,065g/cm³.

11. Procédé selon les revendications 8 à 10, caractérisé par le fait que l'agent matifiant comprend de 0,1 à 1,5% de sphères du copolymère de polyvinylidène et de 2,0 à 4,0% d'un mica enrobé d'une quantité inférieure à 2% de sphères de silice.

12. Procédé selon les revendications 8 à 11, caractérisé par le fait que la base dudit rouge à lèvres comprend de 10 à 50% en poids d'un ester d'acide isononanoïque et de 0,25 à 1,50% d'une silice fumée.

13. Procédé selon les revendications 8 à 12, caractérisé par le fait que ledit agent matifiant comprend de 0,25 à 0,45% en poids de sphères du copolymère de polyvinylidène et de 2,5 à 4,0% en poids d'un mica enrobé d'une quantité inférieure à 2% de sphères de silice.

## Claims

1. Matt lipstick composition, characterized in that it comprises a lipstick base and spheres of a polyvinylidene copolymer in an effective amount giving a matt appearance.

2. Matt lipstick composition according to Claim 1, characterized in that the said spheres of the polyvinylidene copolymer are present in a proportion between 0.1 and 1.5% by weight relative to the lipstick composition.

3. Matt lipstick composition according to Claim 1 or 2, characterized in that the spheres of the polyvinylidene copolymer have a particle size of 5 to 80 µm and a density of 0.01 to 0.065 g/cm³.

4. Matt lipstick composition according to any one of Claims 1 to 3, characterized in that it also comprises, as complementary matt-effect agent, from 2.0 to 4.0% by weight of a mica coated with an amount of less than 2% of silica spheres.

5. Matt lipstick composition according to any one of Claims 1 to 4, characterized in that it also comprises from 10 to 50% by weight of an isononanoic acid ester and from 0.25 to 1.50% by weight of a fumed silica.

6. Matt lipstick composition according to one of Claims 1 to 5, characterized in that it comprises a lipstick base containing from 10 to 50% by weight of an isononanoic acid ester and from 0.25 to 1.50% by weight of fumed silica, as well as from 2.0 to 4.0% by weight of a mica coated with an amount of less than 2% of silica spheres and from 0.1 to 1.5% by weight of spheres of a polyvinylidene copolymer, the said spheres of the polyvinylidene copolymer and the mica coated with silica spheres being present in an amount which is suitable to give a matt appearance.

7. Matt lipstick composition according to Claim 6, characterized in that it comprises from 2.5 to 4.0% by weight of a mica coated with an amount of less than 2% of silica spheres and from 0.25 to 0.45% by weight of a polyvinylidene copolymer.

8. Process for the preparation of a lipstick, characterized in that a matt-effect agent is added to a lipstick base, so as to give a matt appearance, the said matt-effect agent comprising spheres of a polyvinylidene copolymer.

9. Process according to Claim 8, characterized in that the said spheres of the polyvinylidene copolymer are present in an amount between 0.1 and 1.5% by weight, relative to the lipstick composition.

10. Process according to Claim 8 or 9, characterized in that the said spheres of the polyvinylidene copolymer have a particle size of 5 to 80 µm and a density of 0.01 to 0.065 g/cm³.

11. Process according to Claims 8 to 10, characterized in that the matt-effect agent comprises from 0.1 to 1.5% of spheres of the polyvinylidene copolymer and from 2.0 to 4.0% of a mica coated with an amount of less than 2% of silica spheres.

12. Process according to Claims 8 to 11, characterized in that the said lipstick base comprises from 10 to 50% by weight of an isononanoic acid ester and from 0.25 to 1.50% of a fumed silica.

13. Process according to Claims 8 to 12, characterized in that the said matt-effect agent comprises from 0.25 to 0.45% by weight of spheres of the polyvinylidene copolymer and from 2.5 to 4.0% by weight of a mica coated with an amount of less than 2% of silica spheres.

## Patentansprüche

1. Matte Lippenstiftzusammensetzung, dadurch gekennzeichnet, daß sie eine Lippenstiftgrundlage sowie Polyvinylidencopolymerkügelchen in einer Menge enthält, die ein mattes Aussehen verleiht.

2. Matte Lippenstiftzusammensetzung nach Anspruch 1, dadurch gekennzeichnet, daß die Polyvinylidencopolymerkügelchen in einem Mengenanteil zwischen 0,1 und 1,5 Gew.-% in bezug auf die Lippenstiftzusammensetzung vorliegen.

3. Matte Lippenstiftzusammensetzung nach Anspruch 1 oder 2, dadurch gekennzeichnet, daß die Polyvinylidencopolymerkügelchen einen Teilchendurchmesser von 5 bis 80 µm und eine Dichte von 0,01 bis 0,065 g/cm³ besitzen.

4. Matte Lippenstiftzusammensetzung nach einem der Ansprüche 1 bis 3, dadurch gekennzeichnet, daß sie zusätzlich noch als ergänzendes Mattierungsmittel 2 bis 4 Gew.-% eines Glimmers enthält, der in einer Menge von weniger als 2 % mit Kieselerdekügelchen beschichtet worden ist.

5. Matte Lippenstiftzusammensetzung nach einem der Ansprüche 1 bis 4, dadurch gekennzeichnet, daß sie zusätzlich noch 10 bis 50 Gew.-% eines Isononansäureesters sowie 0,25 bis 1,5 Gew.-% flammendispersen Siliziumdioxids enthält.

6. Matte Lippenstiftzusammensetzung nach einem der Ansprüche 1 bis 5, dadurch gekennzeichnet, daß sie eine Lippenstiftgrundlage mit einem Gehalt an 10 bis 50 Gew.-% eines Isononansäureesters sowie 0,25 bis 1,50 % an flammendispersem Siliziumdioxid und außerdem 2,0 bis 4,0 Gew.-% eines Glimmers enthält, der mit einer Menge von weniger als 2 % Kieselerdekügelchen und 0,1 bis 1,5 Gew.-% am Polyvinylidencopolymerkügelchen beschichtet ist, wobei die Polyvinylidencopolymerkügelchen und der mit Kieselerdekügelchen umhüllte Glimmer in einer zur Verleihung des matten Aussehens geeigneten Menge vorhanden sind.

7. Matte Lippenstiftzusammensetzung nach Anspruch 6, dadurch gekennzeichnet, daß sie 2,5 bis 4,0 Gew.-% eines Glimmers enthält, der mit einer Menge von weniger als 2 % Kieselerdekügelchen und 0,25 bis 0,45 Gew.-% Polyvinylidencopolymer beschichtet worden ist.

8. Verfahren zur Herstellung einer Lippenstiftzubereitung, dadurch gekennzeichnet, daß zu einer Lippenstiftgrundlage ein Mattierungsmittel zur Verleihung eines matten Aussehens hinzugefügt wird, wobei das Mattierungsmittel Polyvinylidencopolymerkügelchen enthält.

9. Verfahren nach Anspruch 8, dadurch gekennzeichnet, daß die Polyvinylidencopoylmerkügelchen in einer Menge zwischen 0,1 bis 1,5 Gew.-% in bezug auf die Lippenstiftzusammensetzung vorhanden sind.

10. Verfahren nach Anspruch 8 oder 9, dadurch gekennzeichnet, daß die Polyvinylidencopolymerkügelchen einen durchschnittlichen Teilchendurchmesser von 5 bis 80 µm und eine Dichte von 0,01 bis 0,065g/cm³ aufweisen.

11. Verfahren nach den Ansprüchen 8 bis 10, dadurch gekennzeichnet, daß das Mattierungsmittel zwischen 0,1 und 1,5 % Polyvinylidencopolymerkügelchen und 2,0 bis 4,9 % eines Glimmers enthält, der mit einer Menge von weniger als 2 % Kieselerdekügelchen beschichtet ist.

12. Verfahren nach den Ansprüchen 8 bis 11, dadurch gekennzeichnet, daß die Lippenstiftgrundlage 10 bis 50 Gew.-% eines Isononansäureesters sowie 0,25 bis 1,50 % an flammendispersem Siliziumdioxid aufweist.

13. Verfahren nach den Ansprüchen 8 bis 12, dadurch gekennzeichnet, daß das Mattierungsmittel 0,25 bis 0,45 Gew.-% Polyvinylidencopolymerkügelchen und 2,5 bis 4,0 Gew.-% eines Glimmers enthält, welcher mit einer Menge von weniger als 2 % Kieselerdekügelchen überzogen ist.
